# EUROPÄISCHE PATENTSCHRIFT

(11) **EP 1 995 598 B1**
(45) Veröffentlichungstag und Bekanntmachung des Hinweises auf die Patenterteilung: **30.04.2014**
(21) Anmeldenummer: 07010467.4
(22) Anmeldetag: 25.05.2007
(51) Int. Cl.: G01R 29/08

(54) **Verfahren und kompakte Vorrichtung zum differenzierten Nachweis von Elektrosmog und dessen Analyse**
Method and compact device for differentiated proof of electro smog and its analysis
Procédé et dispositif compact destiné à la vérification différenciée du brouillard chargé en électricité et son analyse

(43) Veröffentlichungstag der Anmeldung: 26.11.2008
(73) Patentinhaber: Lambda: 4 Entwicklungen GmbH, 22767 Hamburg (DE)
(72) Erfinder: Reimann, Rönne, 22769 Hamburg (DE)
(74) Vertreter: Raffay & Fleck

(56) Entgegenhaltungen:
- EP-A- 1 316 806
- US-A1- 2002 021 130
- US-A1- 2006 139 032
- US-A1- 2007 063 905

## Beschreibung

Die vorliegende Erfindung befasst sich mit einem Verfahren und einer kompakten Vorrichtung zum differenzierten Nachweis von Elektrosmog und dessen Analyse.

Durch fortschreitende Nutzung von Funkwellen und Elektrizität ist der Mensch immer stärker elektromagnetischen Feldern ausgesetzt. Dies wird nicht zuletzt durch die massive Aufstellung von Mobilfunkstationen deutlich verstärkt. Der Elektrosmog hat in den vergangenen Jahren deutlich zugenommen.

Bei Elektrosmog handelt es sich um elektromagnetische Strahlung ohne ionisierende Wirkung. Auf Grund der beschränkten Energie eines jeden einzelnen Quants verursacht sie keine direkten Schäden an Molekülen, biologischem Gewebe oder der Erbsubstanz. Bei sehr großer Energiedichte kann sie jedoch über thermische Erhitzung, wie dies beispielsweise bei Mikrowellen angewendet wird, Gewebeschäden verursachen.

Die gebräuchlich mit Elektrosmog bezeichneten Leistungsdichten sind jedoch vergleichsweise niedrig, so dass sie vom menschlichen Körper nicht bewusst wahrgenommen werden. Dies erhöht jedoch ihre Gefährlichkeit, da dadurch Ursachen zahlreicher Erkrankungen im Dunkeln beleiben können und Gegenmaßnahmen häufig unterbleiben. Studien weisen jedoch auf den schädlichen Einfluss von Elektrosmog auf den Organismus hin. Zurzeit laufende, breit angelegte epidemiologische Studien zur Wirkung elektromagnetischer Strahlung werden in der Zukunft weitere Aufschlüsse geben.

Generell kann jedoch von einer zunehmenden Sensibilisierung der Verbraucher in Bezug auf Elektrosmog gesprochen werden und eine weitere Ausdehnung des Interesses an Aufklärung erwartet werden.

Schon jetzt sind zahlreiche Mittel zur Reduzierung des Elektrosmogs erhältlich. So werden beispielsweise Dämmmaterialien zur Reduzierung der in Räumen vorhandenen elektromagnetischen Strahlung angeboten (Abschirmung).

Allerdings ist die Wirkung der Maßnahmen häufig nicht gesichert. Unkenntnis und eine Vielzahl auch kleiner Quellen führen zu nicht ausreichender Reduzierung der Elektrosmogbelastung. Daher besteht der Bedarf an Messgeräten zum Nachweis und Lokalisieren von Elektrosmogquellen.

Da die Umgebung in der der Mensch sich in heutiger Zeit befindet häufig durch eine Vielzahl von elektrischen Geräten, Funkverbindungen sowie Leitungen geprägt ist, ist bei solchen Geräten nicht mehr unbedingt ein breitbandiger Einsatz, sonder vielmehr eine genaue räumliche Auflösung erforderlich. Dafür wären eine geringere Ausdehnung der Messapparatur sowie eine hohe Empfindlichkeit bei möglichst geringem Empfängervolumen von großer Hilfe. Zur Aufspürung der Quellen kann zudem die Kenntnis der Polarisation der elektromagnetischen Strahlung hilfreich sein.

Neben diesem Einsatzbereich besteht auch bei sensiblen elektrischen Geräten selbst häufig die Notwendigkeit, die umgebende Elektrosmogbelastung zu kennen. Gerade bei Geräten, die über elektromagnetische Wellen kommunizieren kann hier auch die Kenntnis der Polarisation des Elektrosmogs dazu dienen, die eigene Kommunikation auf einer mit weniger Elektrosmog belasteten Polarisationsrichtung zu führen. Auch hier ist eine möglichst kompakte Ausführung wünschenswert, um die weiter anhaltende Miniaturisierung elektrischer Bauelemente nicht zu kontrakarieren.

Geräte zum Nachweis von Elektrosmog sind beispielsweise aus der DE 298 10 794 und aus der DE 296 14 412 bekannt. Auch ist aus der US 2006/0139032 ist eine Vorrichtung zur Analyse elektromagnetsicher Strahlung bekannt, bei der unter Verwendung von einander gegenüberliegenden, parallelen, leitenden Platten, vorzugsweise auf den Flächen eines Würfels angeordnet, die Ladungen auf einander gegenüberliegenden Platten durch jeweils ein an jeder Platte angeschlossenes Kabel abgegriffen und differenziell verstärkt und anschließend ausgewertet werden.

Die bekannten Geräte weisen jedoch den Mangel auf, dass mit ihnen auf Grund ihrer Ausdehnung und ihrer einfach ausgestalteten Empfangs- sowie Anzeigemittel eine hohe räumliche Auflösung und somit eine genaue Analyse der vorliegenden Elektrosmogsituation und die Identifizierung einzelner kleiner Quellen nicht möglich ist.

Somit ist es Aufgabe der Erfindung, eine Vorrichtung sowie ein Verfahren anzugeben, dass es ermöglicht, elektromagnetische Strahlung auch bei sehr beschränktem zur Verfügung stehendem Raum zu empfangen sowie gegebenenfalls zu analysieren.

Dies wird gelöst durch eine Vorrichtung nach Anspruch 1 sowie ein Verfahren nach Anspruch 9 unter der Verwendung einer parallelen Anordnung leitender Platten gemäß Anspruch 1 beziehungsweise durch eine solche parallele Anordnung selbst.

Die Unteransprüche geben jeweils Weiterentwicklungen an.

Nach Anspruch 7 weist eine erfindungsgemäße Vorrichtung zum differenzierten Nachweis von Elektrosmog und dessen Analyse eine parallele Anordnung zweier leitender Schichten gemäß einem der Ansprüche 1 bis 6, sowie mindestens einen Verstärker und eine elektronische Verarbeitungseinheit, die über mindestens einen D/A-Wandler mit den Verstärkern, sowie einer Anzeige, einer Schnittstelle und/oder einem Speicher verbunden ist und so eingerichtet ist, dass die Vorrichtung den Wert der gesamten empfangenen Strahlungsleistung anzeigen kann, auf. Dementsprechend werden in einer einfachsten Ausführung die Leistungsentnahmepunkte durch Leistungsentnahmevorrichtungen der ersten parallelen Platte gebündelt auf einen Verstärker geführt. Dessen Ausgang ist über einen digital-analog-Wandler (D/A-Wandler) mit einer elektronischen Verarbeitungseinheit, beispielsweise einem Mikroprozessor, verbunden. Die Verarbeitungseinheit, die auch aus wenigen einfachen Bauteilen, wie Widerständen, Spulen und/oder Kondensatoren bestehen kann, steht wiederum in Kontakt mit einer Anzeige. Denkbar ist es aber auch, auf eine solche Anzeige zu verzichten. Stattdessen oder ergänzend können Schnittstellen oder Speichereinrichtungen vorgesehen werden. Vorzugsweise werden an der Vorrichtung auch Bedienelemente beispielsweise zur Wahl des Verstärkungsfaktors oder sonstiger Parameter vorgesehen.

Bevorzugt ist jedoch jeder Leistungsentnahmepunkt über je eine Leistungsentnahmevorrichtung mit einem eigenen Verstärker verbunden. Diese Verstärker sind bevorzugt wiederum jeweils über einen D/A-Wandler mit der Verarbeitungseinheit verbunden. Denkbar ist jedoch auch, dass die Verarbeitungseinheit nur mit einem D/A-Wandler verbunden ist und dieser nacheinander den Ausgangswert der einzelnen Verstärker ausliest oder eine angesteuerte Umschalteinrichtung zwischen Leistungsentnahmepunkten und einem oder mehreren Verstärkern vorgesehen ist. Darüber hinaus können auch Signale einiger Leistungsentnahmevorrichtung zusammengefasst werden und zusammen jeweils einem Verstärker zugeführt werden, so dass beispielsweise ein Verstärker pro Polarisierungsrichtung vorgesehen ist.

Die parallele Struktur kann beispielsweise auf dem Gehäuse platziert werden, um die Empfangsempfindlichkeit möglichst hoch zu halten, in dem Abschirmung vermieden wird. Bestimmte Einsatzbedingungen können aber auch eine geschützte Platzierung im Gehäuseinneren empfehlenswert machen. Denkbar ist auch eine Anordnung der zwei Platten auf jeweils einer Seite des Gehäuses. Zur Erhöhung der Empfindlichkeit oder zur Schaffung der Möglichkeit, zahlreiche Frequenzen bzw. Frequenzbänder und/oder mehr als zwei Polarisationsrichtungen empfangen zu können, können auch mehrere gleiche oder unterschiedliche parallele Strukturen vorgesehen werden.

Die parallele Anordnung gemäß Anspruch 1 kann für sich genommen ebenfalls eine Erfindung darstellen. Sie zeichnet sich dadurch aus, dass die parallel angeordneten leitenden Schichten, die durch eine nicht leitende Schicht voneinander getrennt sind, so ausgebildet sind, dass eine erste der beiden Schichten in zwei Ausdehnungsrichtungen eine Ausdehnung von etwa der Hälfte der Wellenlänge der zu empfangenen Strahlung aufweist und die zweite Platte in diese beiden Raumrichtungen im Wesentlichen mindestens gleich große oder größere Ausdehnungen aufweist, und dass zwei Leistungsentnahmevorrichtung zur Leistungsentnahme an zwei Leistungsentnahmepunkten aus der ersten Schicht vorgesehen sind. Dementsprechend sind erfindungsgemäß zwei leitende Platten übereinander anzuordnen. Die Platten müssen jedoch nicht zwangsläufig planar und somit koplanar angeordnet sein. Sie können auch leicht gebogen oder Kugeloberflächenausschnitte sein. In einem Solchen Fall ist die Parallelität sozusagen infinitissimal zu gewährleisten. Das heißt, dass der (senkrechte) Abstand zwischen den Platten an jedem Punkt gleich groß sein sollte. Dabei sind an die Parallelität keine zu hohen Anforderungen zu stellen. Je nach Abstand der Platten und deren Größe, sind Winkelabweichungen von der Parallelität von beispielsweise 20° durchaus möglich. Eine solche Abweichung muss nicht über die gesamte Ausdehnung gleich sein. Auch lokal verschiedene Abweichungen sind tolerabel und können größere Werte annehmen. Somit sind auch an die Ebenheit der Platten an sich keine zu großen Anforderungen zu stellen.

Die Ausdehnung der ersten Platte ist so zu wählen, dass sie zwei Kanten mit im Wesentlichen einer Länge, die mit der Hälfte der Wellenlänge der zu empfangenen Strahlung übereinstimmt, aufweist.

Beträgt beispielsweise die Wellenlänge der hauptsächlich zu empfangenen Strahlung 3 cm, wäre eine Kantenlänge von etwa 1,5 cm zu wählen. Dabei ist in der Regel auf die Wellenlänge in der zwischen den Platten liegenden Schicht abzustellen, die durch deren Dielektrizitätskonstante (in der Praxis meist zwischen 3 und 10) deutlich von der Wellenlänge in Luft abweichen kann. Eine solche parallele Anordnung empfängt aber nicht nur Strahlung der Wellenlänge von 12 cm sondern weist eine gewisse Bandbreite auf.

Bei der Wahl des Materials ist neben der für die Wahl der Ausdehnung der Struktur mitverantwortliche Dielektrizitätskonstante auch die Polarisationsfähigkeit des Materials zu berücksichtigen. Denn durch Umpolarisation innerhalb der isolierenden Schicht können beträchtliche Verringerungen der entnehmbaren Leistung auftreten.

Da die Erfindung nicht an quadratische Plattenformen gebunden ist, sondern auch andere mindestens um zwei Linien spiegelsymmetrische Formen, wie beispielsweise Parallelogramme, abgerundete Parallelogramme oder Kreise, möglich sind, ist der Begriff der Kantenlänge so zu verstehen, dass die Kantenlänge des die auf eine Ebene, die über alle Punkte der Platten gemittelt die geringste Winkelabweichung von der Parallelität aufweist, projizierte Platte umschließenden Quadrats der Hälfte der Wellenlänge im Wesentlichen zu entsprechen hat. Das bedeutet, dass in der Platte auch Aussparungen möglich sind, sofern diese die Anforderungen an die Symmetrie gewährleisten.

Die Dicke der Platte ist dabei deutlich geringer als die Kantenlänge zu wählen. Die Dicke bezieht sich auf die Dicke in jeweils einem Punkt der Plattenoberfläche und nicht auf den Abstand von zwei Ebenen, die eine gegebenenfalls gewölbte Platte von oben und untern berühren.

Der (punktuelle) Abstand zwischen den Platten liegt vorzugsweise im Bereich von einigen Prozent der Wellenlänge, kann aber in weiten Bereichen variiert werden. Bei einer zu empfangenen Wellelänge (in der isolierenden Schicht) von 3 cm kommen als Abstand beispielsweise 0,5 mm in Frage. Die Dicke der Platten liegt typischerweise deutlich unter der Größe des Abstandes, kann aber auch vergleichbar groß oder größer gewählt werden.

Als isolierende Schicht kommen zahlreiche Stoffe in Betracht, in einer bevorzugten Ausführung hingegen, wird die isolierende Schicht aus Epoxydharz gebildet. Aber auch eine isolierende Schicht aus Gasen, insbesondere Luft, ist denkbar.

Durch das Vorsehen von zwei Leistungsentnahmevorrichtung an zwei Leistungsentnahmepunkten kann überraschenderweise auf einer vorgegebenen Fläche gegenüber dem Vorsehen von nur einer über Leistungsentnahmevorrichtung an einem Leistungsentnahmepunkt in etwa die im Polarisationsmittel um den Faktor Wurzel-aus-zwei (1,414...) erhöhte Empfangsleistung realisiert werden.

Durch einfallende elektromagnetische Strahlung wir die erste Platte in Dehnungsschwingungen innerhalb der Ebene ihrer Erstreckung (also senkrecht zur Senkrechten auf der Platte) versetzt.

Die entnehmbare Empfangsleistung ist abhängig von der gewählten Lage des jeweiligen Leistungsentnahmepunktes der Leistungsentnahmevorrichtung und der Polarisation der einfallenden elektromagnetischen Strahlung.

Die zweite Platte kann bei gemeinsamer Anordnung mehrer paralleler Empfangsstrukturen großflächig ausgebildet sein und für mehrere erste Platten als zweite Platte dienen.

Besonders vorteilhaft ist die Wahl einer ersten quadratischen, ebenen Platte, da die entnehmbare Leistung von der Fläche der ersten Platte abhängt. Für die Wahl anderer Forman können aber neben der Erhöhung der Bandbreite der Empfangsstruktur zahlreiche Faktoren, wie beispielsweise die Gehäuseform und -größe, sprechen.

Je nach Anwendungsumfeld kann eine indirekte, elektrodynamische Entnahme der Leistung aus den Leistungsentnahmepunkten, also eine elektrodynamische Ankopplung durch Leistungsentnahmevorrichtungen die keinen leitenden Kontakt herstellen gegenüber einer verdrahteten Ankopplung, also einem klassischen Anschluss, Vorteile Aufweisen. Insbesondere können hier Abstände von 10 - 20 % der Wellenlänge gewählt werden. Dies ermöglicht es beispielsweise, die Ankopplung durch ein geschlossenes Gehäuse hindurch zu erreichen. Dies kann insbesondere bei extremen Umweltbedingungen von großem Vorteil sein.

In einer bevorzugten Ausführung der parallelen Anordnung werden die Leistungsentnahmevorrichtungen so angeordnet, dass die Entnahmepunkte jeweils auf einer Mittelsenkrechten unterschiedlicher aneinander angrenzender Kante der ersten Platte liegen. Besonders vorteilhaft ist es, sie auf diesen Mittelsenkrechten randnah zu platzieren. Der Minimale Abstand zum Rand ist jedoch durch die Impedanz des angeschlossenen Leiters und der an diesem Punkt der ersten Platte vorhandenen Schwingungsamplitude begrenzt. Die Bestimmung geeigneter Parameter ist dem Fachmann leicht möglich.

Durch die Platzierung der Leistungsentnahmepunkte auf den Mittelsenkrechten, also auf den Linien, die die erste Platte senkrecht zu ihren Kanten halbiert, wird es möglich, die Empfangsleistung durchschnittlich über alle Polarisationsrichtungen zu maximieren. Dabei sind folgende Fälle linearer Polarisierung zu unterscheiden (die analogen Überlegungen zu anderen Polarisierungen kann der Fachmann analog durchführen, da alle anderen Polarisierungen aus linearen (zeitlich versetzten) Superpositionierungen der hier diskutierten erzeugbar sind): Ist die Strahlung in Flucht mit der Senkrechten auf der Platte polarisiert, das heißt der Polarisationsvektor parallel zu dieser Senkrechten, ist kein Empfang möglich. Liegt eine Polarisierung entlang einer der Mittelsenkrechten vor, wird Leistung nur an der zugehörigen Leistungsentnahmevorrichtung zur Verfügung stehen. Analoges gilt für entlang der anderen Mittelsenkrechten polarisierte elektromagnetische Strahlung. Ist die Strahlung in der Ebene der Plattenausdehnung polarisiert, die Polarisation jedoch mit keiner der Mittelsenkrechten parallel, ist an beiden Leistungsentnahmepunkten durch die Leistungsentnahmevorrichtungen Leistung abzugreifen. Das Verhältnis der an beiden Leistungsentnahmevorrichtungen zur Verfügung stehenden Leistung steht in direktem Verhältnis zu den Winkeln, die die Mittelsenkrechten und der (auf die Ebene der Platte projizierte) Polarisationsvektor bilden.

Dabei ist es überraschend, dass eine erfindungsgemäße parallele Anordnung im Polarisationsmittel die gleiche Leistung liefert wie eine (nur) mit um den Faktor Wurzel-auszwei größeren Kantenlänge zu realisierende Kombination aus zwei entsprechenden parallelen Anordnungen mit jeweils nur einer Leistungsentnahmevorrichtung, deren Orientierungen so gewählt sind, dass die von Ihnen am stärksten empfangbaren Polarisationsrichtungen senkrecht aufeinander stehen. Dadurch ist eine Platz- und Kostenersparnis realisierbar.

Durch die mit der erhöhten Flächenausbeute einhergehende Möglichkeit der Auflösung der empfangenen Leistung nach Polarisationsrichtungen ist es besonders vorteilhaft eine Vorrichtung zum differenzierten Nachweis von Elektrosmog und dessen Analyse gemäß Anspruch 8 so einzurichten, dass die Vorrichtung eine nach der Polarisationsrichtung der empfangenen Strahlung aufgeschlüsselte Anzeige ermöglicht. Dies kann bei der Suche nach der Strahlenquelle und der Verbesserung der Abschirmung ohne Beeinträchtigung der Funktion der abzuschirmenden Geräte (beispielsweise bei Funkverbindungen durch Rundstrahler, wie sie bei WirelessLAN häufig zum Einsatz gelangen und bei denen in stationärer Anordnung jedoch nur wenige Winkelbereiche und Polarisationsrichtungen abzudecken sind) von großer Hilfe sein.

Eine getrennte Auswertung der den beiden Leistungsentnahmepunkten durch die beiden Leistungsentnahmevorrichtungen entnommenen Leistung weist darüber den Vorteil auf, dass eine destruktive Interferenz, die bei bestimmten Polarisationsformen auftreten kann, verhindert wird.

Dies kann besonders gut erreicht werden, wenn jede Leistungsentnahmevorrichtung mit jeweils einem Verstärker verbunden ist. Dabei ist unter einer Leistungsentnahmevorrichtung Vorrichtung zur elektromagnetischen und/oder elektrischen Ankopplung zu verstehen. Aus Kosten und Platzgründen kann durch Umschaltvorrichtungen die Anzahl der Verstärker reduziert werden ohne auf die Möglichkeit der Auflösung nach Polarisationsrichtungen verzichten zu müssen. Dadurch verringert sich jedoch die summierte verwertbare Empfangsleistung.

Unter Schutz soll auch ein Verfahren zum differenzierten Nachweis von Elektrosmog und dessen Analyse gemäß Anspruch 9 gestellt werden, bei dem mindestens eine parallele Anordnung leitender Platten gemäß Anspruch 1 bis 6 zur Gewinnung von Information über elektromagnetische Strahlung auf beschränktem Raum verwendet wird.

Besonders vorteilhaft ist ein solches Verfahren zum differenzierten Nachweis von Elektrosmog und dessen Analyse gemäß Anspruch 10 dann, wenn die aus den Leistungsentnahmepunkten durch die Leistungsentnahmevorrichtungen entnommene Leistung jeweils einem Verstärker zugeführt werden und aus den gewonnen Signalen eine Aufschlüsslung der empfangenen Leistung nach Polarisationsrichtung berechnet und insbesondere dann wenn eine nach Polarisationsrichtung aufgeschlüsselte Anzeige vorgesehen ist.

Weiter Vorteile werden durch die nachfolgende Beschreibung von Ausführungsbeispielen, auf die die Erfindung nicht beschränkt ist, deutlich. Dabei zeigen die Figuren:
- Fig. 1: Schnitt durch eine koplanare Anordnung leitender Platten,
- Fig. 2: Aufsicht auch eine koplanare Anordnung leitender Platten,
- Fig. 3: erfindungsgemäße Vorrichtung und
- Fig. 4: erfindungsgemäße Vorrichtung mit Sensorarm.

Die Figuren sind schematisch und insbesondere nicht maßstabsgetreu. Sie sind nicht beschränkend und zahlreiche weitere Ausführungsbeispiele sind denkbar. Insbesondere lassen sich die Beispiele durch einen Fachmann an die jeweiligen Anforderungen anpassen.

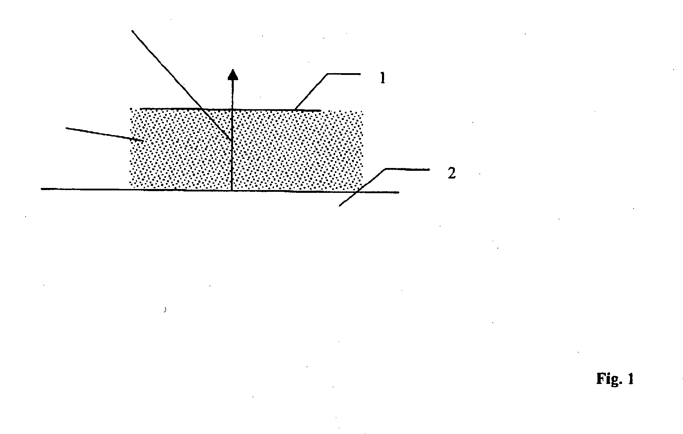

Fig. 1 zeigt einen Schnitt durch eine koplanare Anordnung zweier leitender Platten und 2. Dazwischen ist eine isolierende Schicht 7 zu erkennen. Eingezeichnet ist zum besseren Verständnis der in Bezug genommenen Raumrichtung auch die Senkrechte auf den Platten (3), die senkrecht auf den Richtungen der Ausdehnung 4 der leitenden Platten 1 und 2 steht.

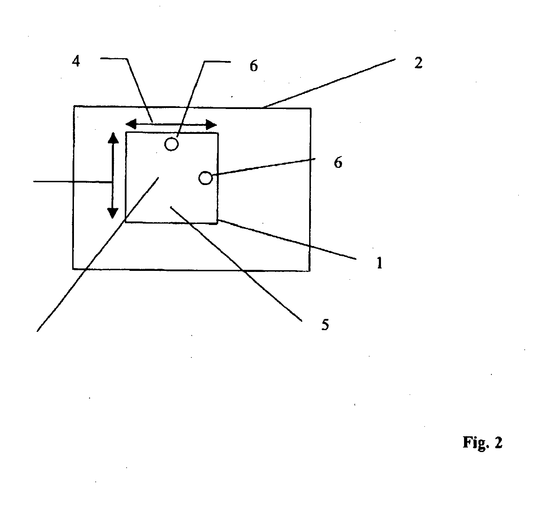

Fig. 2 zeigt eine Aufsicht auf eine solche Anordnung. Die isolierende Schicht ist hier der Größe der ersten leitenden Platte 1 angepasst und somit in dieser Ansicht nicht zu erkennen. Des Weiteren sind die auf die Wellenlänge der zu empfangenen Strahlung in der isolierenden Schicht (gegenüber der Wellelänge in Luft um den Faktor Dielektrizitätskonstante (in diesem Ausführungsbeispiel etwa 4,4) verändert) angepassten Ausdehnungen 4 der ersten leitenden Platte eingezeichnet. Die Kantenlänge der quadratischen leitenden Platte 1 beträgt die Hälfte der Wellenlänge der zu empfangenden Strahlung. Beim Einfall elektromagnetsicher Strahlung, die eine Polarisation außerhalb aller zu ersten leitenden Platte 1 paralleler Ebenen aufweist wird die erste leidende Platte 1 zu Schwingungen angeregt. Diese elektrischen Schwingungen innerhalb der ersten leitenden Platte können über Leistungsentnahmevorrichtungen an den Leistungsentnahmepunkten 6 abgegriffen werden. Eine solche Leistungsentnahmevorrichtung kann beispielsweise durch direkte Kontaktierung der ersten Platte 1 mit einem elektrischen Leiter, über elektromagnetische Kopplung, über Hohlleiter oder ähnliche Ankopplungen realisiert werden. Die Vielzahl der unterschiedlichen Ankopplungsmöglichkeiten macht deutlich, dass ein Leistungsentnahmepunkt 6 einer Leistungsentnahmevorrichtung nicht unbedingt punktförmig sein muss, sondern, beispielsweise bei der Verwendung von Hohlleitern als Leistungsentnahmevorrichtung, auch einen beispielsweise rechteckigen Querschnitt aufweisen kann.

Optimalerweise werden die Leistungsentnahmepunkte 6 auf Mittelsenkrechten aneinander angrenzender Kanten 5 positioniert. Dies maximiert die Empfangsempfindlichkeit und ermöglicht eine Detektion der Polarisationsrichtung der einfallenden elektromagnetischen Strahlung. Je weiter ein Leistungsentnahmepunkt an die Kante der ersten leitenden Platte (also aus der Mitte der Platte heraus) verlegt wird, desto höher ist die zu entnehmende Spannung. Diese Wahl der Leistungsentnahmepunkte ist jedoch durch die empfangene Leistung beschränkt. Somit kann der Leistungsentnahmepunkt 6 sinnvoller Weise nicht immer beliebig weit an eine Kante der ersten leitenden Platte 1 gelegt werden.

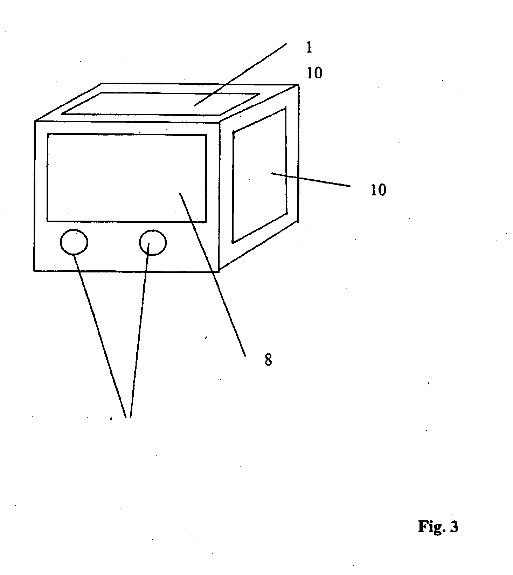

Fig. 3 zeigt eine kompakte Bauweise einer erfindungsgemäßen Vorrichtung. Auf einem die Elektronik umschließenden Gehäuse 12 sind erfindungsgemäße koplanare Anordnungen leitender Platten 10 sowie ein Display 8 und Bedienelemente 9 angeordnet. Dabei kann es sinnvoll sein die koplanaren Anordnungen leitender Platten 10 gegen die im Gehäuse befindliche Elektronik zu schirmen. Durch die senkrecht zueinander stehenden koplanaren Anordnungen leitender Platten 10 ist eine Detektion von beliebig polarisierter elektromagnetischer Strahlung möglich. Auf den verborgenen Seitenflächen können weitere koplanare Anordnungen leitender Platten 10 angebracht werden. Zum Schutz der leitenden Platten kann es auch sinnvoll sein sie innerhalb des Gehäuses 12 zu positionieren.

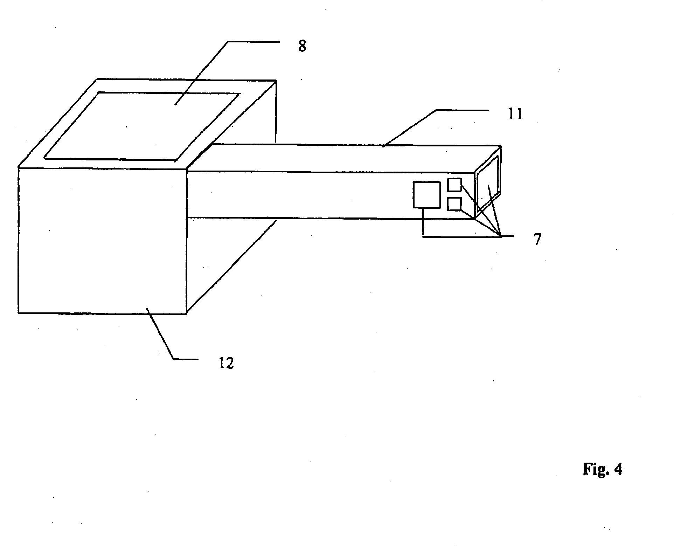

Fig. 4 zeigt eine erfindungsgemäße Vorrichtung mit einem Sensorarm 11, auf dem koplanare Anordnungen leitender Platten 10 in verschiedener Größe angebracht sind. Dies ermöglicht es, elektromagnetische Strahlung in verschiedenen Bändern nachzuweisen. Durch das Anbringen der koplanaren Anordnungen von leitenden Platten 10 auf einem Sensorarm kann die Handhabung der Vorrichtung erleichtert und die räumliche Auflösung verbessert werden. Des Weiteren kann dadurch eine bessere Schirmung von der Geräteelektronik erreicht werden. Allerdings kann es sinnvoll sein, im Sensorarm 11 Vorverstärker unterzubringen, um die Leitungswege zwischen Leistungsentnahmepunkten 6 und Verstärken möglichst kurz zu halten.

Weiter Abwandlungen sind denkbar und durch den Fachmann realisierbar. So können beispielsweise auf dem Gehäuse 12 weiter koplanare Anordnungen leitender Platten 10 positioniert werden oder der Sensorarm ausfahr-, einschieb- oder klappbar ausgeführt werden.

### Bezugszeichenliste

- 1: erste leitende Platte
- 2: zweite leitende Platte
- 3: Senkrechte auf Platten
- 4: Ausdehnung der ersten leitenden Platte in koplanarer Richtung (Hälfte der Wellenlänge)
- 5: Mittelsenkrechte auf Kante der ersten leitenden Platte
- 6: Leistungsentnahmepunkt
- 7: Nicht leitende Schicht
- 8: Display
- 9: Bedienelemente
- 10: Koplanare Anordnung leitender Platten
- 11: Sensorarm
- 12: Gehäuse

## Patentansprüche

1. Parallele Anordnung (10) zweier leitender Platten (1, 2) zum Empfang elektromagnetischer Strahlung mit zwei Leistungsentnahmevorrichtungen, wobei die Parallelität dadurch gegeben ist, dass der Abstand zwischen den Platten an jedem Punkt etwa gleich groß ist, und wobei die leitenden Platten (1, 2) durch eine nicht leitende Schicht (7) getrennt sind und wobei die erste der leitenden Platten (1) in zwei Richtungen eine Ausdehnung (4) von etwa der Hälfte der Wellenlänge der zu empfangenen Strahlung aufweist und eine Spiegelsymmetrie zu zwei Linen aufweist und die zweite leitende Platte (2) in diese beiden Richtungen im Wesentlichen mindestens gleich große oder größere Ausdehnungen aufweist, **dadurch gekennzeichnet, dass** jede der zwei Leistungsentnahmevorrichtungen zur Leistungsentnahme aus je einem von zwei Leistungsentnahmepunkten (6) auf der ersten Platte (1) ausgebildet ist,

2. Parallele Anordnung nach Anspruch 1, **dadurch gekennzeichnet, dass** die erste der leitenden Platten (1) quadratisch ausgebildet ist.

3. Parallele Anordnung nach einem der obigen Ansprüche, **dadurch gekennzeichnet, dass** die Leistungsentnahmepunkte so ausgebildet sind, dass die Leistungsentnahme an den Leistungsentnahmepunkten (6) über eine elektrodynamische Ankopplung erfolgt.

4. Parallele Anordnung nach einem der obigen Ansprüche, **dadurch gekennzeichnet, dass** die Leistungsentnahmepunkte (6) jeweils auf einer Mittelsenkrechten (5) unterschiedlicher aneinander angrenzender Kanten der ersten Platte (1) liegen, insbesondere auf diesen randnah positioniert sind.

5. Parallele Anordnung nach einem der obigen Ansprüche, **dadurch gekennzeichnet, dass** jede Leistungsentnahmevorrichtung mit einem Verstärker verbunden ist.

6. Parallele Anordnung nach einem der Ansprüche 1 bis 4, **dadurch gekennzeichnet, dass** die Leistungsentnahmevorrichtungen mit einer Umschaltungseinrichtung verbunden sind, die mit mindestens einem Verstärker verbunden ist.

7. Vorrichtung zum differenzierten Nachweis von Elektrosmog und dessen Analyse beinhaltend mindestens eine parallele Anordnung (10) zweier leitender Platten (1, 2) gemäß einem der Ansprüche 1 bis 6, sowie mindestens einen Verstärker, vorzugsweise jeweils einen Verstärker pro Leistungsentnahmepunkt (6), wobei jeder der mindestens eine Verstärker mit mindestens einer der Leistungsentnahmevorrichtungen verbunden ist, sowie einer elektronischen Verarbeitungseinheit, die mit einer Anzeige (8), einer Schnittstelle und/oder einem Speicher sowie über mindestens einen D/A-Wandler mit dem mindestens einen Verstärkern verbunden ist und so eingerichtet ist, dass die Vorrichtung den Wert der gesamten empfangenen Strahlungsleistung anzeigen kann.

8. Vorrichtung nach Anspruch 7, **dadurch gekennzeichnet, dass** die Verarbeitungseinheit so eingerichtet ist, dass die Vorrichtung eine nach der Polarisationsrichtung der empfangenen Strahlung aufgeschlüsselte Anzeige ermöglicht.

9. Verfahren zum differenzierten Nachweis von elektromagnetischer Strahlung und dessen Analyse bei dem mindestens eine parallele Anordnung (10) leitender Platten (1, 2) gemäß einem der Ansprüche 1 bis 2 zur Gewinnung von Information über elektromagnetische Strahlung verwendet wird.

10. Verfahren zum differenzierten Nachweis von elektromagnetischer Strahlung und dessen Analyse nach Anspruch 9, **dadurch gekennzeichnet, dass** die aus den Leistungsentnahmepunkten (6) durch die Leistungsentnahmevorrichtungen entnommenen Leistungen jeweils einem Verstärker zugeführt werden und aus den gewonnen Signalen eine Aufschlüsslung der empfangenen Leistung nach Polarisationsrichtung berechnet und insbesondere auch zur Anzeige gebracht wird.

## Claims

1. Parallel arrangement (10) of two conducting plates (1, 2) for receiving electromagnetic radiation comprising two power output devices, wherein the parallelity is given by the fact that the distance between said plates is approximately equal in any point, and wherein the conducting plates (1, 2) are separated by a non-conducting layer (7), and wherein the first of said conducting plates (1) provides an extension (4) in two directions of approximately half of the wave length of the radiation to be received and provides a mirror symmetry against two lines, and the second conducting plate (2) provides in these two directions substantially at least two extensions of the same or of a larger size, **characterized in that** each of the two power output devices for power output is formed by respectively one of said two power output points (6) on the first plate (1).

2. Parallel arrangement according to claim 1, **characterized in that** the first of the conducting plates (1) is provided in a square shape.

3. Parallel arrangement according to one of the preceding claims, **characterized in that** the power output points are formed in such a way that the power output in the power output points (6) is performed through an electro-dynamic coupling.

4. Parallel arrangement according to one of the preceding claims, **characterized in that** the power output points (6) are respectively located on a mid-perpendicular (5) of different edges of said first plate (1), said edges being adjacent with each other, and particularly are located thereon in a position close to the rim.

5. Parallel arrangement according to one of the preceding claims, **characterized in that** each power output device is connected to an amplifier.

6. Parallel arrangement according to one of the claims 1 through 4, **characterized in that** the power output devices are connected to a switching device which is connected to at least one amplifier.

7. Device for the differentiated detection of electro smog and its analysis comprising at least one parallel arrangement (10) of two conducting plates (1, 2) according to one of the claims 1 through 6, and at least one amplifier, preferably one amplifier per power output point (6) respectively, wherein each of the at least one amplifier(s) is connected to at least one of the power output devices and an electronic processing unit which is connected to a display (8), an interface and/or a memory as well as through at least one D/A converter to said at least one amplifier(s), and is configured such that the device can display the value of the complete radiation output received.

8. Device according to claim 7, **characterized in that** the processing unit is configured in such a way that the device facilitates a display itemized according to the polarization direction of the radiation received.

9. Process for the differentiated detection of electromagnetic radiation and its analysis in which at least one parallel arrangement (10) of conducting plates (1, 2) according to one of the claims 1 through 2 is used for the generation of information on electromagnetic radiation.

10. Process for the differentiated detection of electromagnetic radiation and analysis thereof according to claim 9, **characterized in that** the power outputs taken out of the power output points (6) by means of the power output devices are respectively fed to an amplifier and **in that**, from the signals generated, an itemization of the power received is calculated according to the direction of polarization, and is particularly also displayed.

## Revendications

1. Agencement parallèle (10) de deux plaques conductrices (1, 2) en vue de la réception d'ondes électromagnétiques avec deux dispositifs de décharge, étant donné que le parallélisme est assuré par le fait que l'écart entre les plaques est sensiblement le même partout, étant donné que les plaques conductrices (1, 2) sont séparées par une couche électriquement isolante (7) et étant donné que la première plaque conductrice (1) présente une étendue (4) dans les deux directions d'environ la moitié de la longueur d'ondes à réceptionner et une symétrie spéculaire par rapport à deux lignes et que la seconde plaque conductrice (2) présente dans ces deux directions des étendues égales ou plus importantes, **caractérisée en ce que** chacun des deux dispositifs de décharge est formé par un des deux points de décharge (6) sur la première plaque (1).

2. Agencement parallèle selon la revendication 1, **caractérisé en ce que** la première des plaques conductrices (1) présente une forme carrée.

3. Agencement parallèle selon l'une des revendications précédentes, **caractérisé en ce que** les points de décharge sont formés de telle manière que la décharge au niveau des points de décharge (6) s'effectue par un couplage électrodynamique.

4. Agencement parallèle selon l'une des revendications précédentes, **caractérisé en ce que** les points de décharge (6) reposent respectivement sur une des médiatrices (5) de différents bords adjacents de la première plaque (1) et sont en particulier positionnés dans une zone limitrophe du bord.

5. Agencement parallèle selon l'une des revendications précédentes, **caractérisé en ce que** chaque dispositif de décharge est relié à un amplificateur.

6. Agencement parallèle selon l'une des revendications précédentes 1 à 4, **caractérisé en ce que** chaque dispositif de décharge est relié à un dispositif de commutation relié à au moins un amplificateur.

7. Dispositif pour la détection différenciée de la pollution électromagnétique (également nommée *smog électromagnétique* ou *électro-smog)* et l'analyse de cette dernière comprenant au moins un agencement parallèle (10) de deux plaques conductrices (1, 2) conformément à l'une des revendications 1 à 6, ainsi qu'au moins un amplificateur, de préférence un amplificateur par point de décharge (6), étant donné que chaque amplificateur est relié à au moins un dispositif de décharge, ainsi qu'une unité de traitement électronique, qui est relié à un affichage (8), une interface et/ou une mémoire ainsi qu'à au moins un convertisseur D/A relié à au moins un amplificateur et configuré de telle manière que le dispositif peut afficher la valeur de la puissance totale d'ondes réceptionnées.

8. Dispositif selon la revendication 7, **caractérisé en ce que** l'unité de traitement est configurée de telle manière que le dispositif permet un affichage indiquant la direction de polarisation des ondes réceptionnées.

9. Procédé pour la détection différenciée des ondes électromagnétiques et l'analyse de ces dernières qui utilise au moins un agencement parallèle (10) de plaques conductrices (1, 2) conformément à l'une des revendications 1 à 2 pour l'obtention d'informations sur les ondes électromagnétiques.

10. Procédé pour la détection différenciée des ondes électromagnétiques et l'analyse de ces dernières selon la revendication 9, **caractérisé en ce que** les puissances déchargées provenant des dispositifs de décharge transmis par les points de décharge (6) sont acheminées respectivement vers un amplificateur et qu'à partir des signaux enregistrés, une ventilation de la puissance réceptionnée est calculée selon la direction de polarisation et, en particulier, affichée.
